# EUROPÄISCHE PATENTSCHRIFT

(11) **EP 2 396 237 B1**
(45) Veröffentlichungstag und Bekanntmachung des Hinweises auf die Patenterteilung: **19.11.2014**
(21) Anmeldenummer: 10704081.8
(22) Anmeldetag: 12.02.2010
(51) Int. Cl.: B65D 35/30, B65D 81/32, B65D 83/00, A61B 17/00, B65D 51/22

(54) **AUSTRAGVORRICHTUNG MIT TUBE**
DISCHARGE DEVICE WITH TUBE
DISPOSITIF D'EXTRACTION AVEC TUBE

(30) Priorität: 13.02.2009 CH 227092009
(43) Veröffentlichungstag der Anmeldung: 21.12.2011
(73) Patentinhaber: Medmix Systems AG, 6343 Rotkreuz (CH)
(72) Erfinder: GRETER, Andy, CH-6340 Baar (CH); DUBACH, Roger, CH-6343 Rotkreuz (CH)
(74) Vertreter: Detken, Andreas
(86) Internationale Anmeldenummer: PCT/CH2010/000039
(87) Internationale Veröffentlichungsnummer: WO 2010/091527

(56) Entgegenhaltungen:
- EP-A- 0 352 370
- EP-A- 1 762 507
- EP-A2- 0 503 824
- DE-C1- 19 710 878
- US-A- 5 161 715
- US-A1- 2003 050 597

## Beschreibung

### TECHNISCHES GEBIET

Die vorliegende Erfindung betrifft eine Austragvorrichtung zum Austragen eines Produkts aus mindestens einem Behälter.

### STAND DER TECHNIK

In verschiedenen Bereichen der Medizin werden Austragvorrichtungen zum Austragen eines fliessfähigen Produkts aus einem Behälter verwendet, wobei der Behälter mit dem Produkt in eine Kammer der Austragvorrichtung eingesetzt werden kann. Bei dem Behälter kann es sich z.B. um eine Glaskarpule handeln, d.h. um ein zylindrisches Glasgefäss, das an einem distalen Ende durch ein durchstechbares Septum verschlossen ist und in dessen proximales Ende ein verschiebbarer Stopfen eingesetzt ist, der das Gefäss proximal verschliesst. Eine solche Austragvorrichtung kann mit mehreren Kammern für verschiedene Komponenten eines Produktes ausgebildet sein und eine Mischvorrichtung zum Mischen der Komponenten innerhalb der Austragvorrichtung aufweisen. Derartige Vorrichtungen finden für unterschiedliche Aufgaben Anwendung, z.B. zum Austragen und anschliessenden Mischen von unterschiedlichen Komponenten eines Knochenzements oder zum Austragen zweier Komponenten eines Medikaments, das erst unmittelbar vor der Verabreichung durch Mischen der beiden Komponenten hergestellt wird.

Karpulen ermöglichen es, empfindliche Produkte, z.B. Komponenten von Medikamenten oder aggressive Substanzen wie ein Monomer eines Knochenzements, über einen längeren Zeitraum zu lagern. Allerdings haben Behälter in der Form von Karpulen verschiedene Nachteile. So sind Karpulen aufgrund der Verwendung von Glas anfällig gegenüber Stössen und können bei unsachgemässer Handhabung brechen. Karpulen sind ausserdem relativ teuer in der Herstellung. Es ist daher wünschenswert, einen kostengünstigen und bruchsicheren Ersatz für Karpulen bereitzustellen, der es ebenfalls ermöglicht, eine Komponente eines Medikaments oder ein anderes empfindliches Produkt über längere Zeit zu lagern, und hierfür eine entsprechend angepasste Austragvorrichtung anzugeben. Die Austragvorrichtung soll sich dabei speziell für die Verwendung im medizinischen Kontext eignen.

Zur Aufbewahrung von fliessfähigen Produkten oder Komponenten werden in anderen technischen Bereichen oftmals Tuben verwendet. Tuben sind im Allgemeinen mit einem starren Abgabebereich und einem formbaren Wandbereich ausgebildet, wobei die Bereiche aus einheitlichem Material bestehen können, wie z. B. Aluminium. Aluminium eignet sich als Material, da es mit dem Produkt in der Tube keine oder nur sehr geringe Wechselwirkungen eingeht. In dem formbaren Wandbereich ist das Material der Tube derart dünn ausgebildet, dass die Wandung zusammengedrückt werden kann. Bei dem starren Abgabebereich verdeckt das Material der Tube eine Öffnung, die zum Abgeben des Produkts aus der Tube geöffnet, z. B. durchstochen wird. In diesem Bereich weist die Tube häufig einen zylindrischen Anschlussbereich auf, der entlang einer Längsrichtung aus dem Abgabebereich vorsteht und an dem ein Aussengewinde ausgebildet ist. Auf das Aussengewinde ist häufig eine Verschlusskappe aufgeschraubt.

Zum Austragen eines Produkts aus einer Tube wird in der WO 2004/066784 eine Austragvorrichtung mit einem Quetschmechanismus verwendet. Die Austragvorrichtung weist eine Kammer auf, in der die Tube untergebracht und gegen eine axiale Verschiebung entlang der Kammer gesichert wird. Die Abgabeöffnung der Tube ragt aus der Kammer heraus. Innerhalb der Kammer ist eine Walze vorgesehen, welche die Tube beginnend von dem Ende, das der Abgabeöffnung gegenüberliegt, gegen die Innenwand der Kammer zusammenquetscht. Durch axiales Vorrollen der Walze in Richtung der Abgabeöffnung mittels eines Walzenantriebs wird der Tubeninhalt aus der Tube gepresst. Eine ähnliche Austragvorrichtung ist aus der WO 00/10880 bekannt. Bei einem derartigen Ausquetschen von Tuben ist jedoch eine Dosierung des Tubeninhalts schwierig und es kann ein beträchtlicher Rest in der Tube verbleiben. Ferner muss die Tube vor dem Einsetzen in die Austragvorrichtung von Hand geöffnet werden. Ein Mischen des Tubeninhalts mit weiteren Komponenten ist nicht gezeigt.

Zum Öffnen und Entleeren einer Tube ist in der US 2008/0037365 A1 ein Gerät gezeigt, das zum Mischen von Knochenzement dient. Bei diesem Gerät ist eine Mischkammer mit einer Pulverkomponente vorgesehen, die einen Anschluss mit einem Innengewinde zum Anschliessen einer Tube mit einer viskosen Komponente aufweist. In dem Anschluss ist ein vorstehender Dorn vorgesehen, der zylindrisch ausgebildet ist und eine Zuleitung zum Inneren der Mischkammer bilden kann. Die Tube weist ein Anschlussteil auf, das ein Aussengewinde aufweist und durch Verschraubung mit dem Innengewinde an der Mischkammer an diese angeschlossen werden kann. Beim Einschrauben der Tube an den Anschluss der Mischkammer wird die Abdeckung der Abgabeöffnung von dem Dorn durchstochen und es entsteht eine Fluidverbindung zwischen dem Innenvolumen der Tube und dem Inneren der Mischkammer. Anschliessend kann die Mischung aus der Mischkammer ausgetragen werden. Zum Austragen des Produkts aus der Tube und Vermischen mit weiteren Komponenten sind mehrere Handgriffe erforderlich, und die Tube muss manuell ausgepresst werden.

Aus EP 1 762 507 ist eine Kartusche zum Austragen eines fluiden Materials wie z.B. einer Dichtmasse bekannt, die zur Verwendung mit einem Pistolendispenser vorgesehen ist. Die Kartusche weist einen röhrenförmigen Behälter mit darin verschiebbarem Kolben auf. An seinem vorderen Ende ist der Behälter mit einer starren Kappe versehen und weist eine Austrittsöffnung auf. Die Seitenwand des Behälters besteht aus einem dünnen Film. Zum Austragen des Materials wird die Kartusche mit ihrem hinteren Ende am Dispenser gesichert, und der Kolben wird durch einen Ratschenmechanismus schrittweise in die Kartusche eingeschoben. Der Dispenser eignet sich jedoch nicht zur Verwendung mit herkömmlichen Tuben und ist für viele Anwendungsfälle im medizinischen Kontext ungeeignet.

Die DE 197 10 878 beschreibt ein Kartuschensystem mit einer Kartuschenpistole, in welcher eine Kartusche gelagert ist. Durch Betätigen der Kartuschenpistole wird die Kartusche nach vorne zur stirnseitigen Austrittsöffnung der Kartuschenpistole hin geschoben, um die Kartusche mittels einer Öffnungsvorrichtung aufzustechen. Der Kartuscheninhalt wird anschliessend ausgepresst.

Die US 2003/0050597 offenbart ein Kartuschensystem in Form einer Doppelspritze. Ein gleichzeitiges Austragen von zwei Substanzen aus separaten Kartuschen erlaubt zudem die in der EP 0 352 370 gezeigte Austragvorrichtung.

Es ist eine Aufgabe der vorliegenden Erfindung, eine Austragvorrichtung zum Austragen eines Produkts aus einem Behälter zu schaffen, bei der das Produkt zuverlässig ausgetragen wird, das Produkt in einfacher Weise zur Weiterverarbeitung zur Verfügung gestellt wird und nur wenige Handgriffe zur Bereitstellung der Austragvorrichtung mit dem Behälter notwendig sind. Der Behälter soll nicht bereits ausserhalb der Austragvorrichtung geöffnet werden müssen.

### DARSTELLUNG DER ERFINDUNG

Diese Aufgabe wird von der Erfindung durch eine Austragvorrichtung nach Anspruch 1 gelöst. Vorteilhafte Ausgestaltungen und weitere Ausführungsbeispiele gehen aus den abhängigen Ansprüchen hervor.

Nach der vorliegenden Erfindung ist eine Austragvorrichtung zum Austragen eines fluiden Produkts aus einem Behälter mit einer Abgabeöffnung vorgesehen, wobei der Behälter als Tube mit einer formbaren Wandung ausgebildet ist. Die Vorrichtung umfasst ein Gehäuse mit einer Kammer, in der die Tube aufgenommen ist. Die Kammer weist eine Kammeröffnung zum Austragen des Produkts aus der Austragvorrichtung auf. In der Tube ist ein beweglicher Stopfen angeordnet, der an dem der Abgabeöffnung gegenüberliegenden Ende relativ zur Tube verschiebbar angeordnet ist und dieses Ende der Tube verschliesst. Ein Vorschubglied ist an den Stopfen anschliessbar und zum Austragen des fluiden Produkts aus der Tube innerhalb der Tube in Richtung der Abgabeöffnung beweglich. Die Austragvorrichtung ist als Mehrkammerspritze ausgebildet.

Die Tube ist vorzugsweise vollständig in der Kammer aufgenommen. Die Abmessungen der Austragvorrichtung sind vorzugsweise derart gewählt, dass das Gehäuse zwischen zwei Fingern einer Hand ergriffen werden kann. Das Vorschubglied weist dann eine Betätigungsfläche auf, so dass das Vorschubglied durch Druck auf die Betätigungsfläche mit dem Daumen derselben Hand manuell vorschiebbar ist.

Die Abgabeöffnung ist vorzugsweise an einem starren Bereich der Tube vorgesehen, an den sich der formbare Bereich anschliesst, der im wesentlichen zur Aufnahme des Produkts vorgesehen ist. Die Abgabeöffnung der Tube ist mit einer durchstechbaren oder durchtrennbaren Abdeckung verschlossen. Die Tube kann ein Aussengewinde aufweisen, das die Abgabeöffnung umgibt. Dabei ist das Aussengewinde vorzugsweise im starren Bereich der Tube ausgebildet. Das auszutragende Produkt ist zwischen der verschlossenen Öffnung und dem Stopfen vorgesehen. An dem der abgedeckten Öffnung gegenüberliegenden Ende der Tube verschliesst der Stopfen die Tube, wobei der Stopfen in Längsrichtung der Tube verschiebbar ist und mit dem Innenumfang der Tube abschliesst.

Die Tube ist z. B. aus einer Aluminiumlegierung oder aus Fluorpolymeren hergestellt. Sie kann zudem eine behandelte Oberfläche aufweisen, wie etwa eine Beschichtung. Diese Materialien haben sich als besonders geeignet zur Aufbewahrung chemisch aggressiver Komponenten erwiesen. Z. B. sind sie geeignet, um Monomere für Knochenzement ohne Wechselwirkung mit dem Monomer aufzunehmen. Als Abdeckung für die Abgabeöffnung der Tube ist vorzugsweise eine Schicht aus dem Material der Tube vorgesehen, wie es auch für den Wandbereich und den Abgabebereich verwendet wird. Bei einer Tube mit einer derart verschlossenen Abgabeöffnung entsteht nur eine einzige Dichtstelle und zwar zwischen der Tubenwandung und dem Stopfen. Es können aber auch beispielsweise Kunststoff- oder Gummifolien oder Membranen zur Abdeckung verwendet werden. Die Abdeckungen können unterschiedliche Dicken aufweisen, sollten jedoch durchstochen oder durchtrennt werden können.

Die Tubenwandung liegt vorzugsweise unmittelbar am Innenumfang der Kammer an, wodurch die Kammerwand als Stabilisierung der Tubenwandung dient. Ferner wird die Tubenwandung zwischen der Kammerinnenwand und dem Stopfen gehalten. Zusätzlich kann die Tube an ihrem der Abgabeöffnung gegenüberliegenden hinteren Ende ein Rückhalteelement aufweisen, das mit der Kammer derart zusammenwirkt, dass eine Verschiebung des Rückhalteelements in Richtung der Abgabeöffnung verhindert wird. Auf diese Weise wird die Tube in der Kammer zusätzlich stabilisiert. Die Kammer und die Tube sind vorzugsweise zylindrisch ausgebildet. Die Tube kann austauschbar in der Kammer vorgesehen sein, d. h. sobald eine Tube entleert wurde, kann die Tube aus der Austragvorrichtung entnommen werden und es kann eine neue gefüllte Tube eingesetzt werden. Dabei kann das Vorschubglied gemeinsam mit der Tube ausgetauscht werden oder an den Stopfen einer neuen Tube erneut angeschlossen werden. Es ist möglich, eine bereits gefüllte Tube in die Austragvorrichtung einzusetzen, oder es kann eine leere Tube in die Austragvorrichtung eingebracht werden, anschliessend befüllt werden und zuletzt der Stopfen in die Tube eingesetzt werden.

Durch die erfindungsgemässe Abgabevorrichtung mit einer Tube, bzw. ein System aus einer Abgabevorrichtung und einer Tube, können die Vorteile der Verwendung von Tuben zur Aufbewahrung von Produkten oder Produktkomponenten genutzt werden und es steht eine zuverlässige Möglichkeit zur Verfügung, den Tubeninhalt zur weiteren Anwendung zur Verfügung zu stellen. Das Produkt kann unmittelbar verbraucht werden oder es kann zur Weiterverarbeitung, wie etwa einem Mischen mit weiteren Komponenten, eingesetzt werden. Die erfindungsgemässe Austragvorrichtung bietet hierfür eine exakte Dosiermöglichkeit bei einfacher Anwendung. Durch den Einsatz eines Stopfens in der Tube zum Austragen des Tubeninhalts aus der Tube kann mit einem höheren Druck gearbeitet werden als bei den herkömmlichen Quetschverfahren. Es ist nicht zu befürchten, dass ein Teil des Tubeninhalts in der Tube verbleibt. Dabei wird die Tube sicher in der Austragvorrichtung gehalten, zum Einen durch die Kammerwand und zum Anderen durch den Stopfen.

Zwischen der Abgabeöffnung der Tube und der Kammeröffnung ist vorzugsweise eine Verbindungsvorrichtung vorgesehen, die die Kammeröffnung mit der Abgabeöffnung der Tube verbindet. Daher kann das Produkt aus der Tube durch die Verbindungsvorrichtung direkt aus der Austragvorrichtung ausgetragen werden. Es ist aber auch möglich weitere Einrichtungen, wie z. B. eine zweite Produktkammer oder eine Mischeinrichtung mit der Kammeröffnung zu kombinieren.

Vorzugsweise ist die Austragvorrichtung als Mehrkammerspritze ausgebildet und weist wenigstens zwei parallel zueinander angeordnete Kammern auf, wobei in wenigstens einer der Kammern eine Tube wie oben beschrieben vorgesehen ist. Es können aber auch mehr als zwei Kammern parallel nebeneinander angeordnet sein und es kann in jeder Kammer eine Tube vorgesehen werden. Als Vorschubglied kann eine Kolbenstange dienen, die an einem Ende der Kammern, das der Austragöffnung gegenüberliegt, aus der Kammer herausragt. Die Kolbenstange kann manuell entlang der Längsachse der Kammer verschoben werden. Die Vorschubglieder für die zwei oder mehr Kammern sind vorzugsweise an ihrem hinteren, der Abgabeöffnung abgewandten Ende miteinander verbindbar oder dauerhaft miteinander verbunden und weisen eine gemeinsame Betätigungsfläche für den Daumen eines Benutzers auf.

Sofern die Abgabeöffnung der Tube noch verschlossen ist, wenn die Tube in die Austragvorrichtung eingesetzt ist, ist eine Öffnungsvorrichtung in der Austragvorrichtung vorgesehen, die die Abdeckung der Abgabeöffnung der Tube öffnet, z. B. durchtrennt oder durchsticht. Die Öffnungsvorrichtung ist verschieblich relativ zur Tube angeordnet. Die Öffnungsvorrichtung kann an der Tube angebracht werden, bevor diese in die Kammer der Austragvorrichtung eingesetzt wird. Alternativ ist es auch möglich, innerhalb der Kammer der Austragvorrichtung eine Öffnungsvorrichtung vorzusehen, welche die Tube z. B. beim Einsetzen in die Kammer durchsticht. Natürlich ist es grundsätzlich auch möglich, die Tube vor dem Einsetzen in die Kammer zu öffnen.

Die Tube ist beweglich in der Kammer der Austragvorrichtung gelagert und es ist ein Betätigungselement zum Vorschub der Tube relativ zur Kammer in Richtung der Austragöffnung vorgesehen. Das Betätigungselement ist mit dem Vorschubglied für den Stopfen identisch und greift zum Vorschub der Tube in der Kammer der Austragvorrichtung vorzugsweise unmittelbar an dem Stopfen in der Tube an. Die Kraftübertragung zum Vorschub der verschlossenen Tube relativ zur Kammer erfolgt in diesem Fall über das inkompressible Produkt. Grundsätzlich ist es aber auch möglich, dass das Betätigungselement zunächst an einem Teil der Tube angreift, solange die Tubenöffnung verschlossen ist, um die Tube vorzuschieben, und nach dem Öffnen der Tubenöffnung derart umgekoppelt wird, dass das Betätigungselement am Stopfen der Tube angreift, um das Produkt aus der Tube auszutragen.

Die Öffnungsvorrichtung weist ein Stechelement auf, das zum Durchstechen oder Durchtrennen der Abdeckung der Tubenöffnung bei einem Vorschub der Tube relativ zum Stechelement vorgesehen ist. Das Stechelement kann beispielsweise als Kanüle oder als Dorn mit einem Durchgang, bzw. einer Durchgangsleitung vorgesehen sein, die in einem Halter angeordnet sind, der im Bereich der abgedeckten Öffnung der Tube auf die Tube aufgesetzt werden kann. Wird das Stechelement relativ zur Tube vorgeschoben, durchsticht die Kanüle oder der Dorn die Abdeckung der Tubenöffnung, so dass ein Zugang zum Tubeninneren entsteht.

Das Stechelement ist vorzugsweise derart an der Tube angeordnet, dass es in einer ersten Position gemeinsam mit der Tube relativ zur Kammer verschiebbar ist und in einer zweiten Position in Vorschubrichtung an einem Anschlag der Kammer anschlägt, womit es relativ zur Kammer feststeht und relativ zur Tube verschiebbar ist. Demnach wird das Stechelement gemeinsam mit der Tube relativ zur Kammer der Austragvorrichtung in Vorschubrichtung verschoben, wenn das Betätigungselement auf die Tube oder den Stopfen wirkt. Sobald das Stechelement an dem Anschlag der Kammer anstösst, wird es an einem weiteren Vorschub gehindert, so dass die Vorschubkraft allein auf die Tube wirkt und diese relativ zum Stechelement und zur Kammer vorschiebt. Dabei wird die Kanüle oder der Dorn des Stechelements auf die Abdeckung der Tubenöffnung zu bewegt, bis es diese durchsticht, bzw. durchtrennt. An dem Stechelement oder auch an der Kammer ist ein weiterer Anschlag für die Tube vorgesehen, an dem die Tube anschlägt, sobald das Stechelement die Abdeckung der Tubenöffnung durchstochen hat, so dass auch die Tube relativ zur Kammer feststeht und die Vorschubkraft des Betätigungselements in Form des Vorschubglieds allein auf den Stopfen in der Tube wirkt, so dass das Produkt aus der Tube durch die Öffnung, bzw. durch die Kanüle ausgetragen werden kann. Vorzugsweise kommt der Ausgang des Durchgangs des Stechelements, durch welchen das Produkt aus der Tube, bzw. aus dem Stechelement, ausgetragen wird, gegenüber der Austragöffnung der Kammer zu liegen, so dass das Produkt aus der Austragvorrichtung vollständig ausgetragen werden kann. Vorzugsweise ist eine Abdichtung zwischen der Austragöffnung der Kammer und dem Stechelement derart vorgesehen, dass der Übergang zwischen dem Stechelement und der Austragöffnung nach aussen abgedichtet ist, sobald das Stechelement an dem Anschlag in der Kammer anschlägt. Die Öffnungsvorrichtung, bzw. das Stechelement, kann somit als Verbindungsvorrichtung dienen, die die Kammeröffnung der Austragvorrichtung mit der Abgabeöffnung der Tube verbindet.

Wie oben beschrieben, ist das Stechelement vorzugsweise beweglich an dem Ende der Tube mit der abgedeckten Öffnung angeordnet. Dabei kommt vorzugsweise das Stechelement in der ersten Position vor der Abdeckung und der zweiten Position, in der das Stechelement relativ zur Tube verschoben ist, in einer Stellung zu liegen, in der die Abdeckung der Öffnung durchstochen ist. Somit kann vorteilhafterweise das Stechelement an der Tube angeordnet werden, bevor die Tube in die Kammer der Austragvorrichtung eingesetzt wird. Hierfür können an dem Stechelement beispielsweise Schnapparme vorgesehen sein, mit welchen das Stechelement beweglich an der Tube befestigt wird.

Vorzugsweise umfasst die Öffnungsvorrichtung eine Führungseinrichtung zum Führen des Stechelements von der ersten Position in die zweite Position. Die Führungseinrichtung kann dabei unmittelbar an der Tube vorgesehen sein. Es ist jedoch auch möglich, die Führungseinrichtung an einem Führungselement vorzusehen, das auf die Tube an dem Ende mit der Abdeckung aufsetzbar ist, wobei das Stechelement dann an dem Führungselement angeordnet werden kann. Das Führungselement kann beispielsweise auf ein Gewinde aufgeschraubt werden, das an dem starren Bereich der Tube um die Abgabeöffnung vorgesehen ist. Es ist aber auch möglich, eine andere Verbindung, wie etwa eine Schnappverbindung oder eine Bajonettverbindung, zwischen dem Führungselement und der Tube vorzusehen.

Die Führungseinrichtung kann beispielsweise als zylindrisches Führungselement ausgebildet sein, das ein erstes Positionierungsmittel und ein zweites Positionierungsmittel, verteilt entlang der Zylinderachse aufweist, die zur Positionierung des Stechelements an der Tube dienen. Die Positionierungsmittel können z. B. als Rillen, Nuten, Kanten oder Vorsprünge am Aussenumfang des Führungselements vorgesehen sein. Dabei ist das erste Positionierungsmittel weiter entfernt von der Abdeckung der Tubenöffnung vorgesehen als das zweite Positionierungsmittel. Der Abstand zwischen dem ersten und dem zweiten Positionierungsmittel ist auf die Wegstrecke abgestimmt, die erforderlich ist, damit das Stechelement die Abdeckung der Tubenöffnung durchstechen kann.

Das Stechelement kann mit den Schnapparmen in der ersten Position z. B. in eine erste Rille eingreifen und durch den Vorschub der Tube aus dieser Eingriffsposition gelöst werden und soweit verschoben werden, bis die Schnapparme in eine zweite Rille eingreifen. Das Zusammenwirken der Rillen und der Schnapparme kann auch als Anschlag für den Vorschub der Tube in der Kammer wirken, so dass diese an einem weiteren Vorschieben gehindert wird.

Das Stechelement kann z. B. eine scheibenförmige Basis mit einem zentralen Durchgang aufweisen, die als Halter für die Kanüle oder den Dorn und die Befestigungseinrichtung zur Befestigung an der Tube, wie die Schnapparme dient. An der Basis kann die Kanüle oder der Dorn zentral angeordnet werden und die Schnapparme können beispielsweise entlang des Umfangs der Basis vorgesehen sein, so dass sie das Führungselement oder die Tube im Bereich der Öffnung umgreifen können. Die Schnapparme können nach innen ragende Vorsprünge aufweisen und sind derart elastisch vorgespannt, dass sie in die Positionierungsmittel der Führungseinrichtung einschnappen, wenn das Stechelement auf die Führungseinrichtung oder die Tube aufgeschoben wird. Dabei können die Vorsprünge der Schnapparme in die Rillen der Positionierungsmittel eingreifen. Um das Lösen aus der Verschnappung zu erleichtern, können schräg zur Längsache verlaufende schräge Flächen an den nach innen ragenden Vorsprüngen der Schnapparme vorgesehen sein, entlang derer die Schnapparme geführt werden, sobald eine Vorschubkraft sie in Richtung der Tube bewegt. Die Schnapparme werden dabei aufgespreizt und solange entlang des Aussenumfangs des Führungselements oder der Tube verschoben, bis sie in die zweite Rille des Positionierungsmittels der Führungseinrichtung eingreifen.

Gemäss einer bevorzugten Ausführungsform der vorliegenden Erfindung ist die Austragvorrichtung in Form einer Doppelkammerspritze vorgesehen. In einer der Kammern ist eine Tube vorgesehen, die eine erste Komponente, z. B. ein Lösungsmittel aufweist, und in der anderen Kammer ist eine zweite Komponente, z. B. eine Knochenzementkomponente, untergebracht. Für die Doppelkammerspritze ist ein Doppelkolben vorgesehen, dessen jeweiliger Kolben in eine der beiden Kammern der Doppelkammerspritze hineinragt und einen Stopfen in der Kammer, bzw. in der Tube vorschiebt. Der Doppelkolben weist zwei Kolbenstangen auf, die an ihrem hinteren Ende miteinander verbunden sind und eine Betätigungsfläche für den Daumen eines Benutzers bilden. Am vorderen Ende, das dem Ende mit dem Doppelkolben gegenüberliegt, weist jede Kammer der Doppelspritze eine Austragöffnung auf. Über der Austragöffnung kann eine Austragdüse, vorzugsweise eine Mischdüse vorgesehen sein, in welche die erste und die zweite Komponente durch Vorschub des Doppelkolbens ausgetragen werden können. Durch fortgesetzten Vorschub des Doppelkolbens werden die beiden Komponenten auch in der Austragdüse vorgeschoben und im Fall einer Mischdüse z. B. zu einem fertig anwendbaren Knochenzement miteinander vermischt. Die Düse weist eine Öffnung auf, aus welcher die erzeugte Mischung aus der Austragdüse austritt.

Die Länge der Kolbenstangen des Doppelkolbens, welche in die jeweilige Kammer hineinragen, ist derart abgestimmt, dass bevor eine Komponente aus einer der Kammern ausgetragen wird, die Abdeckung der Öffnung der Tube mittels des Stechelements durchstochen und somit geöffnet wird.

Hierfür kann auf der Tube über dem starren Bereich mit der abgedeckten Öffnung zunächst das Führungselement aufgesetzt, beispielsweise aufgeschraubt, sein. Auf der Aussenumfangsfläche des Führungselements ist die Öffnungsvorrichtung z. B. in Form des Stechelements angeordnet. Das Stechelement greift mit den Schnapparmen in eine erste, vordere Rille auf dem Aussenumfang des Führungselements ein. Die Einheit aus Tube, Führungselement und Stechelement wird in die Kammer der Doppelkammerspritze eingesetzt. Anschliessend wird der Doppelkolben in die beiden Kammern eingesetzt. Durch Vorschub des Doppelkolbens wirkt der Kolben auf den Stopfen der Tube, wodurch die Tube relativ zur Kammer in Richtung der Austragöffnung der Kammer vorgeschoben wird. Die Tube wird solange vorgeschoben, bis das Stechelement an einem Anschlag in Form des Bodens der Kammer anschlägt. Der Doppelkolben wird weiterhin vorgeschoben, wodurch die Tube gemeinsam mit dem Führungselement relativ zum Stechelement weiterverschoben wird, da das Stechelement an dem Anschlag der Kammer gehalten wird. Das Stechelement wird von der ersten Position, in der es vor der Abdeckung der Tubenöffnung zu liegen kommt, in die zweite Position verschoben, in der es die Abdeckung durchsticht. Hierfür weist das Führungselement eine Öffnung auf, durch die das Stechelement hindurch auf die Abdeckung geführt werden kann.

In dieser zweiten Position ist eine Fluidverbindung zwischen dem Inneren der Tube und der Austragöffnung der Kammer hergestellt, so dass die Komponente aus der Tube durch die Tubenöffnung und die Austragöffnung der Kammer in die Austragdüse ausgetragen werden kann. Durch weiteres Vorschieben des Doppelkolbens wird nun die erste Komponente aus der Tube und gleichzeitig auch die zweite Komponente aus der zweiten Kammer der Doppelkammerspritze ausgetragen und in die Mischdüse ausgeschüttet.

Grundsätzlich kann an Stelle der Doppelkammerspritze auch eine Mehrkammerspritze mit mehr als zwei Kammern verwendet werden, wobei der Kolben ebenfalls als Mehrfachkolben ausgebildet ist.

Gemäss der vorliegenden Erfindung kann ein System aus einer Austragvorrichtung und einer Tube bereitgestellt werden, wie sie oben beschrieben sind. Dabei ist die Kammer der Austragvorrichtung auf die Ausmasse der Tube abgestimmt, so dass der Tubeninhalt durch Vorschub des Stopfens innerhalb der Tube mittels eines Vorschubglieds ermöglicht wird.

### KURZE BESCHREIBUNG DER ZEICHNUNGEN

Eine bevorzugte Ausführungsform der Erfindung wird im Folgenden anhand der Zeichnungen dargestellt, die lediglich zur Erläuterung dienen und nicht einschränkend auszulegen sind. Aus den Zeichnungen offenbar werdende Merkmale der Erfindung sollen einzeln und in jeder Kombination als zur Offenbarung der Erfindung gehörend betrachtet werden. In der Zeichnung stellen dar:
- Fig. 1:: eine Explosionsdarstellung einer Austragvorrichtung nach der vorliegenden Erfindung,
- Fig. 2:: einen teilweisen Längsschnitt durch eine zusammengesetzte Austragvorrichtung nach Figur 1,
- Fig. 3:: einen Längsschnitt durch eine Detailansicht der Austragvorrichtung in einer ersten Position,
- Fig. 4:: einen Längsschnitt durch die Detailansicht aus Figur 3 in einer teilweise vorgeschobenen Position,
- Fig. 5:: einen Längsschnitt durch eine Detailansicht der Austragvorrichtung in einer zweiten Position und
- Fig. 6:: eine dreidimensionale Darstellung der Detailansicht nach Figur 3.

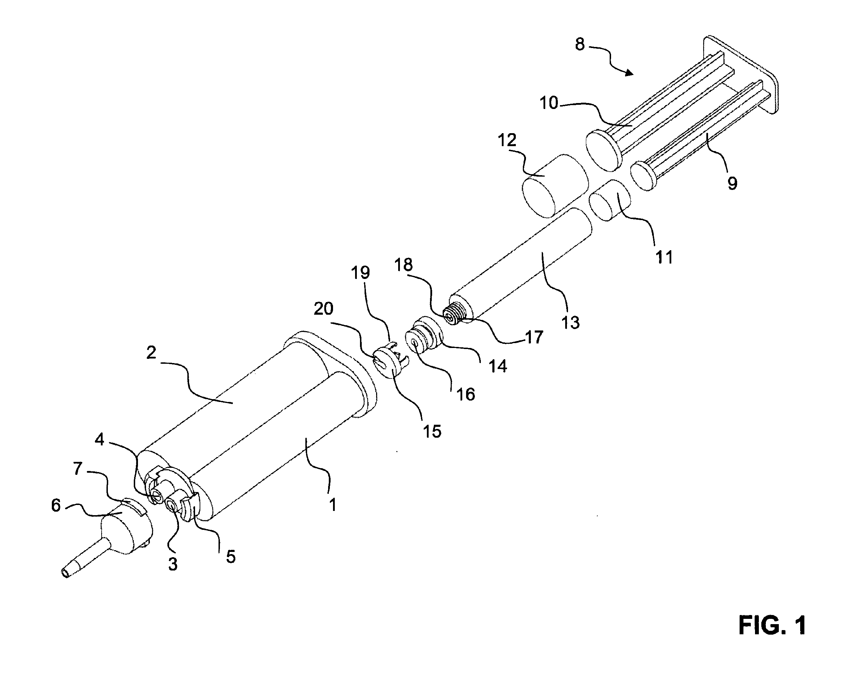

In Figur 1 ist eine Explosionsdarstellung einer Austragvorrichtung gemäss einer Ausführungsform der vorliegenden Erfindung gezeigt. Die Austragvorrichtung weist die Form einer Doppelkammerspritze auf, die eine erste Kammer 1 und eine zweite Kammer 2 aufweist, die zylindrisch ausgebildet sind und parallel nebeneinander angeordnet sind. An einem Ende weisen die beiden Kammern je eine Austragöffnung 3 und 4 auf, die eine Fluidverbindung in das Innere der Kammern 1 und 2 herstellen. Die Austragöffnungen 3 und 4 sind als zylinderartige Fortsätze parallel nebeneinander angeordnet. Neben den Austragöffnungen ist eine Befestigungseinrichtung 5 vorgesehen, die zur Befestigung einer Austragdüse 6 dient. Die Befestigungseinrichtung 5 kann beispielsweise durch Haltenuten gebildet werden, in welche an der Austragdüse 6 angebrachte Halteleisten 7 eingreifen können. Vorzugsweise ist die Befestigungseinrichtung lösbar ausgebildet, so dass die Austragdüse 6 über den Austragöffnungen 3 und 4 ausgetauscht werden kann. Die Befestigungseinrichtung kann beispielsweise als Bajonettverbindung vorgesehen sein. An dem Ende der ersten und zweiten Kammer 1 und 2, das den Austragöffnungen 3 und 4 gegenüberliegt, ist ein Vorschubglied in Form eines Doppelkolbens 8 mit einer ersten Kolbenstange 9 und einer zweiten Kolbenstange 10 vorgesehen.

In der ersten Kammer 1 wird eine Tube 13 untergebracht, die einen vorderen starren Bereich mit einer verschlossenen Öffnung 18 aufweist, der zylindrisch ausgebildet ist und auf dessen Aussenumfang ein Aussengewinde 17 vorgesehen ist. An den starren Bereich schliesst sich ein zylindrischer Bereich mit einer formbaren Wandung an. Ein erster Stopfen 11 ist für die Tube 13 und ein zweiter Stopfen 12 ist für die zweite Kammer 2 vorgesehen. Auf die Stopfen 11 und 12 wirken jeweils die Kolbenstangen 9 und 10 des Doppelkolbens 8. Der Doppelkolben dient somit zum Vorschieben der Stopfen.

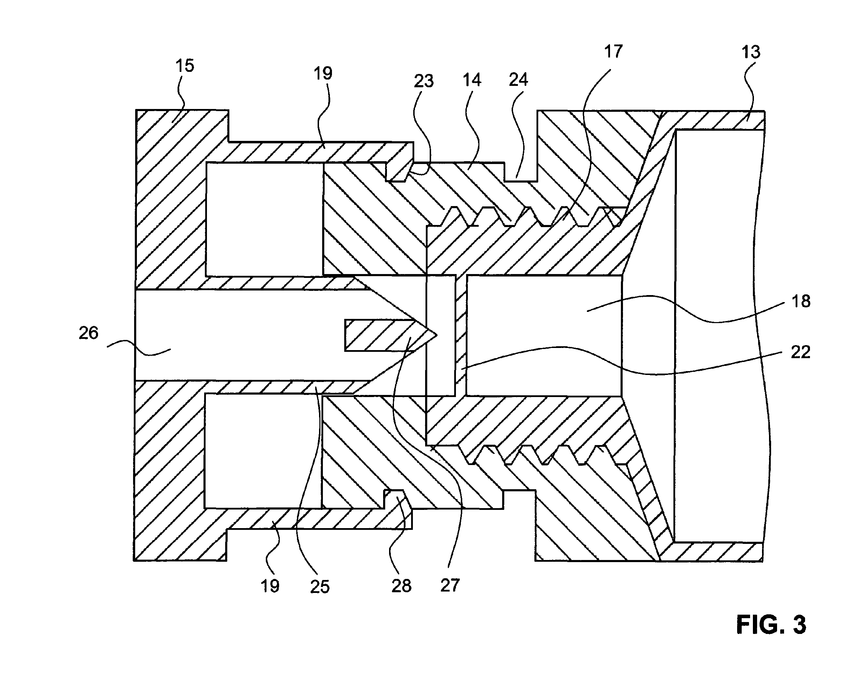

An der Tube 13 ist ein Führungselement 14 und eine Öffnungsvorrichtung in Form eines Stechelements 15 vorgesehen. Das Führungselement 14 ist zylinderartig ausgebildet und weist einen inneren Durchgang 16 mit einem Innengewinde auf. Das Führungselement 14 kann mit dem Innengewinde auf das Aussengewinde 17 der Tube 13 aufgeschraubt werden. Der Durchgang 16 schliesst sich somit an die Öffnung 18 der Tube 13 an. Das Stechelement 15 weist drei Schnapparme 19 auf, die sich in Längsrichtung der Tube 13 in Richtung des Führungselements 14 erstrecken. Das Stechelement 15 wird in den Figuren 3 bis 6 genauer beschrieben.

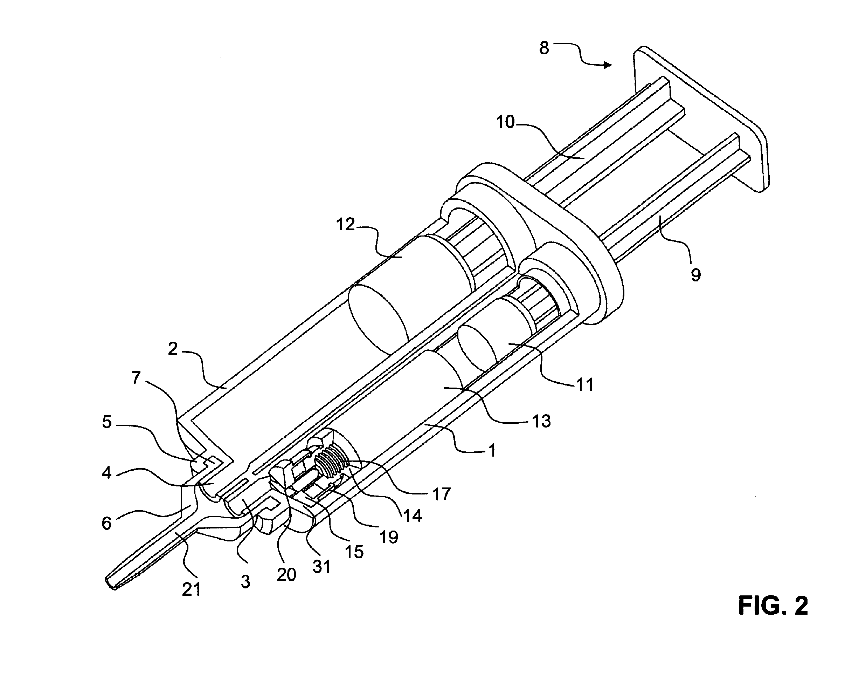

In Figur 2 ist die Austragvorrichtung nach Figur 1 in zusammengesetztem Zustand gezeigt. Dabei ist das Führungselement 14 auf das Gewinde 17 der Tube 13 aufgeschraubt und das Stechelement 15 ist mit den Schnapparmen 19 auf das Führungselement 14 aufgeschnappt. Die Einheit aus Tube 13, Führungselement 14 und Stechelement 15 ist in die erste Kammer 1 der Doppelkammerspritze eingesetzt. Der erste Stopfen 11 verschliesst das Ende der Tube 13, das der Öffnung 18 gegenüberliegt. Der erste Stopfen 11 ist verschieblich in der Tube 13 gelagert und schliesst die Tube 13 flüssigkeitsdicht ab. Der zweite Stopfen 12 ist in der zweiten Kammer 2 verschieblich gelagert und schliesst die zweite Kammer 2 flüssigkeitsdicht ab. Zwischen der Öffnung 18 der Tube 13 und dem Stopfen 11 ist eine erste Komponente einer Mischsubstanz und zwischen der Austrittöffnung 4 und dem zweiten Stopfen 12 ist in der zweiten Kammer 2 eine zweite Komponente der Mischsubstanz gelagert. An die Stopfen 11 und 12 schliessen sich die erste Kolbenstange 9 und die zweite Kolbenstange 10 des Doppelkolbens 8 derart an, dass sie in die erste Kammer 1 und die zweite Kammer 2 mit einem Ende hineinragen und mit dem gegenüberliegenden Ende aus den Kammern hervorstehen. Durch Betätigung, bzw. Vorschub, des Doppelkolbens 8 in die Kammern 1 und 2 hinein können die Stopfen 11 und 12 in Richtung der Austragöffnungen 3 und 4 in Längsrichtung der Kammern bewegt werden. Ferner kann die Tube 13 innerhalb der ersten Kammer mit der Kolbenstange 9 axial verschoben werden, so dass die Kolbenstange 9 auch als Betätigungselement zum Vorschieben der Tube innerhalb der Kammer dient.

Wie in den Figuren 1 und 2 ersichtlich, weist das Stechelement 15 eine Basisscheibe auf, wobei die Schnapparme 19 im wesentlichen senkrecht von dieser Basisscheibe abragen. Es sind drei Schnapparme 19 vorgesehen, die am Rand der Basisscheibe gleichmässig am Umfang der Basisscheibe verteilt sind. Ist das Stechelement 15 auf das Führungselement 14 aufgesetzt, kommen die Schnapparme 19 am Aussenumfang des Führungselements 14 zu liegen und werden dabei elastisch radial aufgeweitet. Durch die elastische Vorspannung der Schnapparme 19 wird das Stechelement 15 auf dem Führungselement 14 gehalten. Die Basisscheibe des Stechelements 15 weist in ihrem Zentrum einen Durchgang auf, der in einen Kanal 20 mündet, der als vertiefte Rille ausgebildet ist und vom Zentrum radial zum äusseren Rand der Scheibe verläuft. Wie in Figur 2 ersichtlich, ist das Stechelement 15 derart an der Tube 13 angeordnet, dass der Kanal 20 in die Austragöffnung 3 mündet, wenn die Tube 13 in die erste Kammer 1 eingesetzt ist. Dadurch ergibt sich eine Fluidverbindung von dem Durchgang 16 des Führungselements 16 zum Kanal 20 des Stechelements 15 bis zur Austragöffnung 3 der Kammer 1 aus. Die Austragdüse 6 ist mit ihren Halteleisten 7 in der Befestigungsseinrichtung 5 befestigt. Die beiden Austragöffnungen 3 und 4 münden somit in einen Austragkanal 21 der Austragdüse 6.

In Figur 3 ist ein Detaillängsschnitt durch das Stechelement 15, das Führungselement 14 und das Ende der Tube 13 mit der Öffnung 18 gezeigt. Die Öffnung 18 der Tube 13 ist durch eine Abdeckung 22 verschlossen, die durchtrennt, bzw. durchstochen werden kann. Das Führungselement 14 ist auf dem Aussengewinde 17 der Tube aufgeschraubt. Das Führungselement 14 weist auf seinem Aussenumfang als Positionierungsmittel eine erste Führungsrille 23 und in Längsrichtung versetzt dazu eine zweite Führungsrille 24 auf, die sich rings um den Umfang des Führungselements erstrecken. In dem gezeigten Ausführungsbeispiel kommt die erste Führungsrille 23 des Führungselements 14 in Längsrichtung der Tube 13 vor der Abdeckung 22 und die zweite Führungsrille 24 kommt hinter der Abdeckung 22 zu liegen.

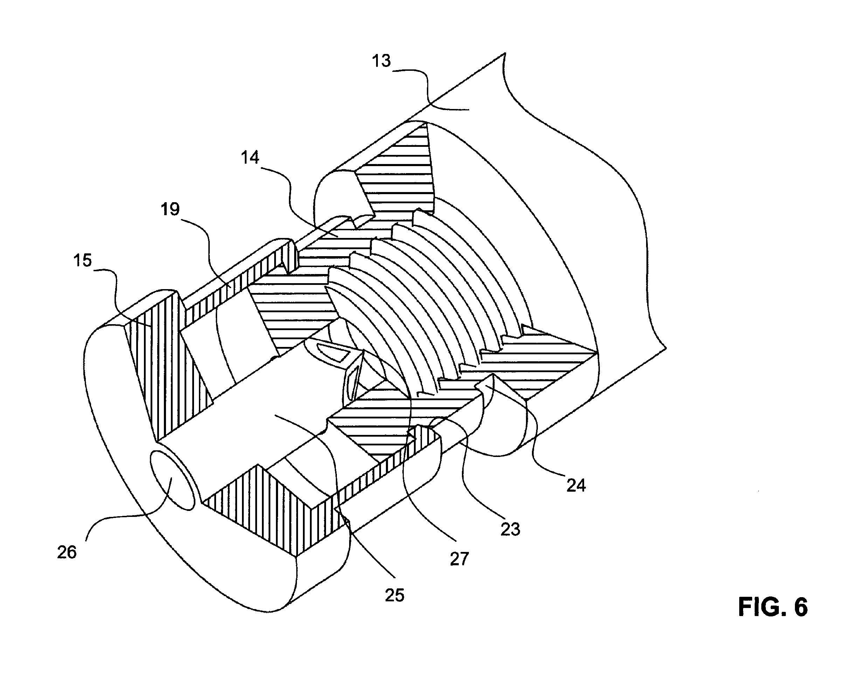

Das Stechelement 15 weist an seinem äusseren Rand die Schnapparme 19 auf. Im Zentrum des Stechelements 15 ist ein länglicher Dorn 25 vorgesehen, der im wesentlichen senkrecht von der Basisscheibe des Stechelements 15 in Richtung der Tube 13 absteht. Der Dorn 25 weist eine Durchgangsleitung 26 auf. An einem Ende des Dorns, das in Richtung der Tube 13 orientiert ist, weist der Dorn im Zentrum eine Stechspitze 27 auf, die zum Durchstechen bzw. Durchtrennen der Abdeckung 22 der Tubenöffnung 18 dient. Die Ausführung der Stechspitze wird in Figur 6 genauer erläutert.

In Figur 3 ist die Einheit aus Tube 13, Führungselement 14 und Stechelement 15 in einer ersten Position gezeigt, die einer Ausgangsposition entspricht. In der Ausgangsposition kommt die Spitze 27 des Dorns 25 kurz vor der Abdeckung 22 zu liegen. In dieser Position kann die Tube über längere Zeit gelagert werden. Die Schnapparme 19 des Stechelements 15 sind in Längsrichtung über das Führungselement 14 geschoben und schnappen mit nach innen gerichteten Vorsprüngen 28 in die erste Führungsrille 23 des Führungselements 14 ein. Das Stechelement 15 wird in dieser Schnappposition sicher auf dem Führungselement 14 gehalten und kann ohne weitere Krafteinwirkung nicht in Richtung der Abdeckung 22 bewegt werden.

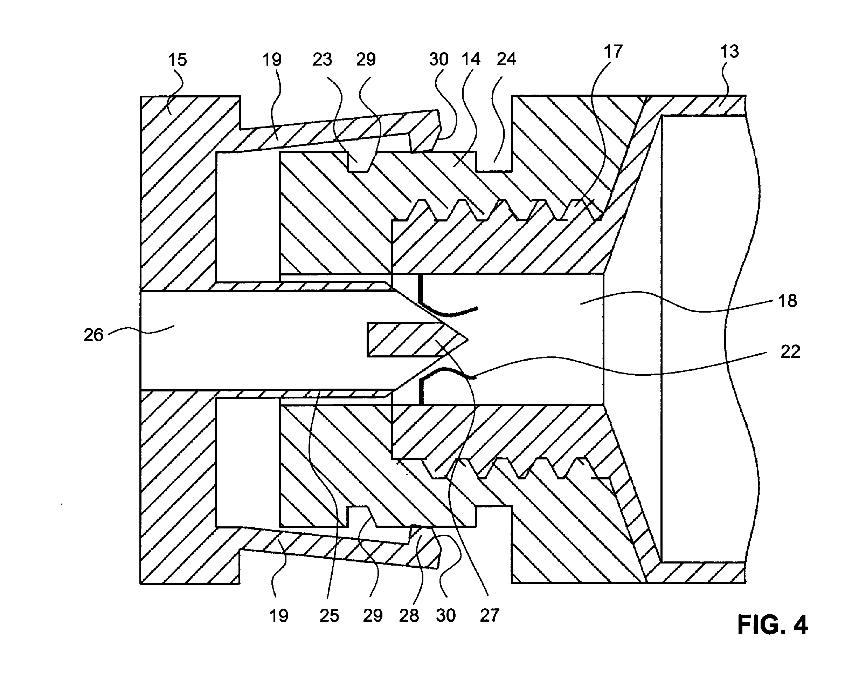

In Figur 4 ist die Einheit aus Tube 13, Führungselement 14 und Stechelement 15 in einer Übergangsposition gezeigt. In der Übergangsposition wurde die Tube 13 mit dem darauf fest angebrachten Führungselement 14 in Längsrichtung relativ zum Stechelement 15 auf das Stechelement zu verschoben. Dabei wurden die Schnapparme 19 radial nach aussen aufgespreizt und aus der ersten Führungsrille 23 herausbewegt, so dass sie auf dem Aussenumfang des Führungselements 14 zu liegen kommen. Die erste Führungsrille 23 weist hierfür eine schräge Fläche 29 auf. Die erste Führungsrille 23 ist somit nach aussen zum Umfang des Führungselements 14 aufgeweitet. Die Vorsprünge 28 der Schnapparme 19 weisen eine zur schrägen Fläche 29 korrespondierende schräge Fläche 30 auf. Der Vorsprung 28 läuft somit nach innen in Richtung des Führungselements 14 verjüngend zu. In Figur 3 kommen die schrägen Flächen 29 der ersten Führungsrille 23 und die schrägen Flächen 30 der Vorsprünge 28 der Schnapparme 19 aufeinander zu liegen. Wird auf die Tube 13 mit dem Führungselement 14 eine Kraft in Richtung des Stechelements 15 ausgeübt, gleiten die schrägen Flächen an einander ab und spreizen dabei die Schnapparme 19 auf, wie in Figur 4 ersichtlich ist. Durch die Relativbewegung zwischen der Tube 13 und dem Stechelement 15 wurde der Dorn 25 des Stechelements 15 in Richtung der Abdeckung 22 bewegt und hat diese durchtrennt. Dabei wurde der Dorn 25 in die Öffnung 18 der Tube 13 soweit eingeführt, dass die Stechspitze 27 die Abdeckung 22 durchstochen hat.

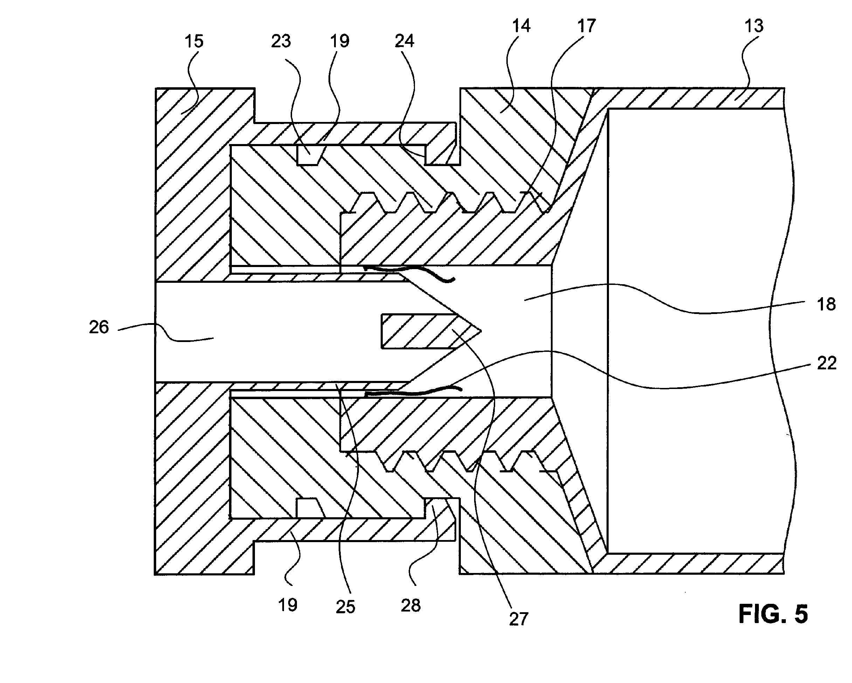

In Figur 5 ist die Einheit aus Tube 13, Führungselement 14 und Stechelement 15 in einer zweiten Position gezeigt, in der das Stechelement 15 relativ zur Tube 13 längs verschoben ist und die Abdeckung 22 durchstochen ist. Die zweite Position entspricht einer Durchstechposition. Im Vergleich zur Position gemäss Figur 4 ist die Tube 13 mit dem Führungselement 14 weiter in Richtung des Stechelements 15 verschoben. Dabei gleiten die Vorsprünge 28 der Schnapparme 19 des Stechelements 15 auf dem Aussenumfang des Führungselements 14 in Richtung der Tube 13 bis sie in die zweite Führungsrille 24 einschnappen. Das Stechelement 15 ist in dieser Position fest an dem Führungselement 14 angebracht. Die Flächen der Vorsprünge 28 und der zweiten Führungsrille 24, die in Längsrichtung aufeinander zu liegen kommen verlaufen im Wesentlichen senkrecht zur Längsrichtung des Führungselements. Wird versucht, das Stechelement 15 von dem Führungselement 14 abzuziehen, werden diese Flächen gegeneinander gedrückt, aber die Schnapparme 19 werden nicht radial nach aussen aufgeweitet. In dieser Position kommt das Stechelement 15 derart auf dem Führungselement 14 zu liegen, dass die Fluidverbindung zwischen der Öffnung 18 der Tube 13 und der Durchgangsleitung 26 des Stechelements 15 flüssigkeitsdicht ist. Die Frontseite des Führungselements 14 kommt dabei auf der Basisscheibe des Stechelements 15 zu liegen und der Innenumfang des Durchgang 16 des Führungselements 14 kommt am Aussenumfang des Dorn 25 zu liegen. Zwischen den Flächen, die aufeinander zu liegen kommen, können beispielsweise Dichtmittel, wie etwa eine Gummibeschichtung oder ein Gummiring, vorgesehen werden.

In Figur 5 ist eine Fluidverbindung zwischen dem Inneren der Tube 13 entlang der Öffnung 18 zur Durchgangsleitung 26 und zum Kanal 20 (nicht gezeigt in Figur 5) hergestellt und bildet somit eine Verbindungsvorrichtung zwischen der Kammeröffnung 3 und der Abgabeöffnung 18 der Tube 13. Die erste Komponente der Mischsubstanz kann somit aus der Tube 13 ausgetragen werden.

In Figur 6 ist eine dreidimensionale Ansicht mit einem Teilschnitt durch die Einheit aus Tube 13, Führungselement 14 und Stechelement 15 gezeigt. In dieser Ansicht ist die Einheit in der ersten Position gezeigt, in welcher die Schnapparme 19 des Stechelements 15 in der ersten Führungsrille 23 des Führungselements 14 eingeschnappt sind. Der Dorn 25 des Stechelements 15 ragt zentral in Richtung der Tube 13 hervor. Die Stechspitze 27 wird durch spitz nach innen zulaufende Stege gebildet, die vom Aussenumfang des Dorns 25 bis zum Mittelpunkt der Durchgangsleitung 26 hervorstehen. Zwischen den einzelnen Stegen und der Umfangswand verbleiben Durchgänge in das Innere zur Durchgangsleitung 26. Die Stege sind an ihrer Aussenseite kantig ausgebildet, so dass sie im Mittelpunkt des Dorns die Stechspitze 27 ausbilden.

Der Ablauf der Anwendung einer Austragvorrichtung nach der vorliegenden Erfindung kann an Figur 2 erläutert werden. In Figur 2 befindet sich die Austragvorrichtung in Form der Doppelkammerspritze in der ersten Position, der Ausgangsposition. Die erste Kolbenstange 9 schliesst sich an den ersten Stopfen 11 in der Tube 13 an. In der Tube 13 befindet sich zwischen der Öffnung 18, die durch die Abdeckung 22 verschlossen ist, und dem ersten Stopfen 11 die erste Komponente der Mischsubstanz. Zur Herstellung der Mischung mit der Doppelkammerspritze kann der Spritzenkörper mit der ersten Kammer 1 und der zweiten Kammer 2 beispielsweise mit den Fingern ergriffen werden, während der Daumen auf dem Doppelkolben 8 zu liegen kommt. Somit kann der Doppelkolben 8 relativ zu den Kammern der Doppelkammerspritze in diese Kammern hineinverschoben werden. Dabei wird eine Vorschubkraft auf den ersten Stopfen 11 ausgeübt, die über die erste Komponente der Mischsubstanz auf die Tube 13 übertragen wird, so dass die Tube 13 relativ zur ersten Kammer 1 in Richtung der Austragöffnung 2 verschoben wird. In Figur 2 ist die Tube 13 bereits soweit vorgeschoben, dass das Stechelement 15 an einer Bodenwand 31 der ersten Kammer 1 anstösst. Die Bodenwand 31 bildet einen Anschlag für den Vorschub der Einheit aus Tube 13, Führungselement 14 und Stechelement 15. Bei einem weiteren Vorschieben des Doppelkolbens 8 wirkt somit die Vorschubkraft auf die Schnapparme 19 des Stechelements 15, welche das Stechelement 15 am Führungselement 14 halten. Wie in den Figuren 4 und 5 beschrieben, werden die Schnapparme 19 durch die Vorschubkraft entlang der schrägen Flächen 29 und 30 radial nach aussen aufgespreizt, so dass die Tube 13 und das Führungselement 14 relativ zum Stechelement in Richtung der Bodenwand 31, bzw. der Austragöffnung 3 vorgeschoben werden können. Die Tube 13 wird solange relativ zum Stechelement 15 verschoben, bis die Schnapparme 19 in die zweite Führungsrille 24 des Führungselements 14 einschnappen. Gleichzeitig stösst die Frontseite des Führungselements 14 gegen den scheibenförmigen Bereich des Stechelements 15. Bei dieser Vorschubbewegung wird die Abdeckung 22 der Öffnung 18 der Tube 13 von der Stechspitze 27 des Dorns 25 des Stechelements 15 durchtrennt.

Die Austragvorrichtung befindet sich nun in der zweiten Position, in der eine Fluidverbindung zwischen dem Inneren der ersten Kammer 1 und der Austragöffnung 3, bzw. dem Austragkanal 21 der Austragdüse 6, ausgebildet ist. Durch ein weiteres Vorschieben des Doppelkolbens 8 wird der erste Stopfen 11 in der Tube 13 und der zweite Stopfen 12 in die zweite Kammer 2 vorgeschoben, so dass gleichzeitig aus der ersten und der zweiten Kammer die erste und zweite Komponente der Mischsubstanz durch die Austragöffnungen 3 und 4 in die Austragdüse 6 entleert werden. In der Austragdüse 6 werden die Komponenten mit einander vermischt. Hierfür ist es möglich, in der Austragdüse 6 zusätzlich Mischeinrichtungen vorzusehen. Durch fortgesetztes Vorschieben des Doppelkolbens 8 wird die Mischung durch den Austragkanal 21 aus der Austragdüse 6 aus der Austragvorrichtung ausgetragen.

Beim Vorschub des Stopfens 11 in der Tube 13 wird der Stopfen relativ zur formbaren Wandung der Tube verschoben, wobei der Innenumfang der Kammer 1 am Aussenumfang der Tube 13 zu liegen kommt und die Tube 13 gegen eine Verformung oder ein Einknicken stützt.

Der Öffnungsmechanismus kann bei einer alternativen Ausführungsform auch durch eine in der Kammer befestigte Kanüle oder dergleichen realisiert werden. Die Tube kann auch vor dem Einsetzen in die Kammer 1 der Austragvorrichtung geöffnet werden, so dass durch Vorschub des Vorschubglieds in Form des Doppelkolbens 8 der Tubeninhalt unmittelbar ausgetragen werden kann. Eine Fluidverbindung wird dabei z. B. durch Anpressen der Tube gegen den Kammerboden hergestellt, wobei die Abgabeöffnung der Tube und die Austragöffnung der Kammer aneinander anschliessen. Der Öffnungsmechanismus, wie er oben beschrieben wurde, ist nicht nur bei Tuben, sondern auch bei anderen Behältern, die eine verschlossene oder abgedeckte Öffnung aufweisen, vorteilhaft einsetzbar. Beispielsweise können auch Kartuschen oder der gleichen mit einem solchen Öffnungsmechanismus geöffnet werden.

**BEZUGSZEICHENLISTE**

| | | | |
|---|---|---|---|
| 1 | erste Kammer | 17 | Aussengewinde |
| 2 | zweite Kammer | 18 | Öffnung Behälter |
| 3 | Austragöffnung 1. Kammer | 9 | Schnapparme |
| 4 | Austragöffnung 2. Kammer | 20 | Kanal |
| 5 | Befestigungseinrichtung | 21 | Austragkanal |
| 6 | Austragdüse | 22 | Abdeckung |
| 7 | Halteleisten | 23 | erste Führungsrille |
| 8 | Doppelkolben | 24 | zweite Führungsrille |
| 9 | erste Kolbenstange | 25 | Dorn |
| 10 | zweite Kolbenstange | 26 | Durchgangsleitung |
| 11 | erster Stopfen | 27 | Stechspitze |
| 12 | zweiter Stopfen | 28 | Vorsprung |
| 13 | Tube | 29 | schräge Fläche Führungsrille |
| 14 | Führungselement | 30 | schräge Fläche Vorsprung |
| 15 | Öffnungsvorrichtung, Stechelement | | |
| | | 31 | Bodenwand |
| 16 | Durchgang Führungselement | | |

## Patentansprüche

1. Austragvorrichtung in Form einer Mehrfachspritze zum Austragen eines fluiden, ersten Produkts aus mindestens einem ersten Behälter, aufweisend:
ein Gehäuse mit mindestens einer ersten Kammer (1), die eine erste Kammeröffnung (3) aufweist und in der der erste Behälter aufgenommen ist, sowie mit mindestens einer zweiten Kammer (2), die eine zweite Kammeröffnung (4) aufweist, und in der ein zweites Produkt aufgenommen ist;
ein bewegliches erstes Vorschubglied (9) zum Austragen des ersten Produkts; und
ein in der zweiten Kammer (2) beweglich angeordnetes zweites Vorschubglied (10);
wobei das erste und das zweite Vorschubglied (9, 10) an einem hinteren, der ersten und zweiten Kammeröffnung abgewandten Ende miteinander verbunden sind,
wobei der erste Behälter die Form einer Tube (13) mit einer Abgabeöffnung (18) und einer formbaren Wandung aufweist,
wobei in der Tube (13) ein beweglicher Stopfen (11) angeordnet ist, der an dem der Abgabeöffnung (18) gegenüberliegenden Ende angeordnet ist und dieses Ende der Tube (13) verschliesst,
wobei das erste Vorschubglied (9) an den Stopfen (11) anschliessbar ist und zum Austragen des fluiden, ersten Produkts aus der Tube (13) innerhalb der Tube (13) in Richtung der Abgabeöffnung (18) beweglich ist,
wobei die Abgabeöffnung (18) der Tube (13) durch eine durchstechbare Abdeckung (22) verschlossen ist,
wobei in der Austragvorrichtung eine relativ zur Tube (13) verschiebliche Öffnungsvorrichtung (15) mit einem Stechelement (15) zum Durchstechen der Abdeckung (22) vorgesehen ist,
wobei die Tube (13) derart beweglich in der ersten Kammer (1) der Austragvorrichtung angeordnet ist, dass das Stechelement (15) die Abdeckung bei einer Bewegung der Tube (13) in Richtung der ersten Abgabeöffnung durchsticht,
und wobei das erste und das zweite Vorschubglied (9, 10) derart dimensioniert sind, dass beim gemeinsamen Vorschieben der Vorschubglieder (9, 10) das erste Vorschubglied (9) die Tube (13) in Richtung der ersten Abgabeöffnung bewegt und dadurch das Stechelement (15) die Abdeckung (22) der Tube (13) durchsticht, bevor das zweite Vorschubglied (10) das zweite Produkt aus der zweiten Kammer (2) ausstösst.

2. Austragvorrichtung nach Anspruch 1, wobei das Gehäuse dazu ausgebildet ist, zwischen den Fingern einer Hand eines Benutzers ergriffen zu werden, und wobei am ersten Vorschubglied (9) eine Betätigungsfläche ausgebildet ist, die derart angeordnet ist, dass sie mit dem Daumen der selben Hand, mit der das Gehäuse ergriffen wird, betätigbar ist, um das erste Vorschubglied (9) zum Austragen des ersten Produkts manuell vorzuschieben.

3. Austragvorrichtung nach Anspruch 1 oder 2, , wobei an der Tube (13) ein Aussengewinde (17) ausgebildet ist, das die Abgabeöffnung (18) umgibt.

4. Austragvorrichtung nach einem der vorhergehenden Ansprüche, **dadurch gekennzeichnet, dass** eine Verbindungsvorrichtung (15) vorgesehen ist, die die erste Kammeröffnung (3) mit der Abgabeöffnung (18) der Tube (13) verbindet.

5. Austragvorrichtung nach einem der vorhergehenden Ansprüche, **dadurch gekennzeichnet, dass** die Öffnungsvorrichtung (15) in einer ersten Position gemeinsam mit der Tube (13) relativ zur Kammer (1) verschiebbar ist und in einer zweiten Position in Vorschubrichtung an einem Anschlag (31) der Kammer (1) anschlägt, wobei sie relativ zur Kammer (1) feststeht und relativ zur Tube (13) verschiebbar ist.

6. Austragvorrichtung nach einem der vorhergehenden Ansprüche, **dadurch gekennzeichnet, dass** die Öffnungsvorrichtung (15) eine innere Durchgangsleitung (26) aufweist, die als Verbindungsvorrichtung dient.

7. Austragvorrichtung nach einem der vorhergehenden Ansprüche, **dadurch gekennzeichnet, dass** eine Führungseinrichtung (14) zum Führen der Öffnungsvorrichtung (15) von einer ersten Position in eine zweite Position relativ zur Tube (13) vorgesehen ist, wobei die Führungseinrichtung (14) ein Führungselement aufweist, das auf die Tube (13) an dem Ende mit der Abdeckung aufschraubbar ist, wobei die Öffnungsvorrichtung (15) an dem Führungselement angeordnet ist.

## Claims

1. A discharge device in the form of a multiple syringe for discharging a fluid, first product from at least one first container, comprising:
a housing having at least one first chamber (1) which has a first chamber opening (3) and in which the first container is accommodated, and having at least one second chamber (2) which has a second chamber opening (4) and in which a second product is accommodated;
a movable first advancing member (9) for discharging the first product; and
a second advancing member (10) which is arranged movably in the second chamber (2);
the first and the second advancing members (9, 10) being connected to each other at a rear end facing away from the first and second chamber opening,
wherein the first container is in the form of a tube (13) with a dispensing opening (18) and a deformable wall,
wherein a movable stopper (11) is arranged in the tube (13), said stopper being arranged at the end opposite the dispensing opening (18) and closing said end of the tube (13),
wherein the first advancing member (9) is adapted to be joined to the stopper (11) and is movable within the tube (13) in the direction of the dispensing opening (18) in order to discharge the fluid, first product from the tube (13),
wherein the dispensing opening (18) of the tube (13) is closed by a pierceable covering (22),
wherein, in the discharge device, an opening device (15) which is displaceable relative to the tube (13) is provided, with a puncturing element (15) for piercing the covering (22),
wherein the tube (13) is arranged movably in the first chamber (1) of the discharge device in such a manner that the puncturing element (15) pierces the covering upon movement of the tube (13) in the direction of the first dispensing opening,
and wherein the first and the second advancing member (9, 10) are dimensioned in such a manner that, upon joint advancing of the advancing members (9, 10), the first advancing member (9) moves the tube (13) in the direction of the first dispensing opening, and as a result, the puncturing element (15) pierces the covering (22) of the tube (13) before the second advancing member (10) ejects the second product from the second chamber (2).

2. The discharge device as claimed in claim 1, wherein the housing is designed to be grasped between the fingers of a hand of a user, and wherein an actuating surface is formed on the first advancing member (9), said actuating surface being arranged in such a manner that it can be actuated with the thumb of the same hand with which the housing is grasped, in order to manually advance the first advancing member (9) to discharge the first product.

3. The discharge device as claimed in claim 1 or 2, wherein an external thread (17) which surrounds the dispensing opening (18) is formed on the tube (13).

4. The discharge device as claimed in one of the preceding claims, **characterized in that** a connecting device (15) is provided, the connecting device connecting the first chamber opening (3) to the dispensing opening (18) of the tube (13).

5. The discharge device as claimed in one of the preceding claims, **characterized in that** the opening device (15), in a first position, is adapted to be advanced together with the tube (13) relative to the chamber (1) and, in a second position, abuts in the advancing direction against a stop (31) of the chamber (1), in which case said opening device is stationary relative to the chamber (1) and displaceable relative to the tube (13) .

6. The discharge device as claimed in one of the preceding claims, **characterized in that** the opening device (15) has an inner passage line (26) which serves as the connecting device.

7. The discharge device as claimed in one of the preceding claims, **characterized in that** a guide means (14) is provided for guiding the opening device (15) from a first position into a second position relative to the tube (13), the guide means (14) having a guide element which is adapted to be screwed onto the tube (13) at the end with the covering, and the opening device (15) being arranged on the guide element.

## Revendications

1. Dispositif d'extraction sous la forme d'une seringue multiple pour extraire un premier produit fluide d'au moins un premier récipient, présentant:
un boîtier comprenant au moins une première chambre (1) qui présente une première ouverture de chambre (3) et dans laquelle est reçu le premier récipient, ainsi qu'au moins une deuxième chambre (2) qui présente une deuxième ouverture de chambre (4) et dans laquelle est reçu un deuxième produit;
un premier organe d'avance mobile (9) pour extraire le premier produit; et
un deuxième organe d'avance (10) disposé de manière mobile dans la deuxième chambre (2);
le premier et le deuxième organe d'avance (9, 10) étant connectés l'un à l'autre au niveau d'une extrémité arrière opposée à la première et à la deuxième ouverture de chambre,
le premier récipient présentant la forme d'un tube (13) avec une ouverture d'écoulement (18) et une paroi formable,
un bouchon mobile (11) étant disposé dans le tube (13), lequel est disposé au niveau de l'extrémité opposée à l'ouverture d'écoulement (18) et fermant cette extrémité du tube (13),
le premier organe d'avance (9) pouvant être raccordé au bouchon (11) et, pour l'extraction du premier produit fluide hors du tube (13), pouvant être déplacé à l'intérieur du tube (13) dans la direction de l'ouverture d'écoulement (18),
l'ouverture d'écoulement (18) du tube (13) étant fermée par un recouvrement (22) pouvant être percé,
un dispositif d'ouverture (15) déplaçable par rapport au tube (13) avec un élément de poinçon (15) pour percer le recouvrement (22) étant prévu dans le dispositif d'extraction,
le tube (13) étant disposé de manière mobile dans la première chambre (1) du dispositif d'extraction de telle sorte que l'élément de poinçon (15) perce le recouvrement dans le cas d'un déplacement du tube (13) dans la direction de la première ouverture d'écoulement,
et le premier et le deuxième organe d'avance (9, 10) étant dimensionnés de telle sorte que lors d'une avance commune des organes d'avance (9, 10), le premier organe d'avance (9) déplace le tube (13) dans la direction de la première ouverture d'écoulement, et que de ce fait l'élément de poinçon (15) perce le recouvrement (22) du tube (13), avant que le deuxième organe d'avance (10) n'éjecte le deuxième produit hors de la deuxième chambre (2).

2. Dispositif d'extraction selon la revendication 1, dans lequel le boîtier est réalisé pour pouvoir être saisi entre les doigts d'une main d'un utilisateur, et dans lequel une surface d'actionnement est réalisée au niveau du premier organe d'avance (9), laquelle est disposée de telle sorte qu'elle puisse être actionnée avec le pouce de la main qui saisit le boîtier, afin de faire avancer manuellement le premier organe d'avance (9) pour extraire le premier produit.

3. Dispositif d'extraction selon la revendication 1 ou 2, dans lequel un filetage extérieur (17) est réalisé sur le tube (13), lequel entoure l'ouverture d'écoulement (18).

4. Dispositif d'extraction selon l'une quelconque des revendications précédentes, **caractérisé en ce qu'**un dispositif de liaison (15) est prévu, lequel relie la première ouverture de chambre (3) à l'ouverture d'écoulement (18) du tube (13).

5. Dispositif d'extraction selon l'une quelconque des revendications précédentes, **caractérisé en ce que** le dispositif d'ouverture (15) peut être déplacé dans une première position conjointement avec le tube (13) par rapport à la chambre (1) et, dans une deuxième position, bute dans la direction d'avance contre une butée (31) de la chambre (1), en étant fixe par rapport à la chambre (1) et déplaçable par rapport au tube (13).

6. Dispositif d'extraction selon l'une quelconque des revendications précédentes, **caractérisé en ce que** le dispositif d'ouverture (15) présente une conduite de passage interne (26) qui sert de dispositif de liaison.

7. Dispositif d'extraction selon l'une quelconque des revendications précédentes, **caractérisé en ce qu'**un système de guidage (14) est prévu pour guider le dispositif d'ouverture (15) d'une première position dans une deuxième position par rapport au tube (13), le système de guidage (14) présentant un élément de guidage qui peut être vissé sur le tube (13) à l'extrémité présentant le recouvrement, le dispositif d'ouverture (15) étant disposé sur l'élément de guidage.
